# EUROPÄISCHE PATENTSCHRIFT

(11) **EP 2 242 698 B1**
(45) Veröffentlichungstag und Bekanntmachung des Hinweises auf die Patenterteilung: **03.08.2011**
(21) Anmeldenummer: 09710279.2
(22) Anmeldetag: 21.01.2009
(51) Int. Cl.: B65D 51/00, A61J 1/14

(54) **VERSCHLUSSKAPPE**
CLOSURE CAP
BOUCHON DE FERMETURE

(30) Priorität: 15.02.2008 DE 102008009418
(43) Veröffentlichungstag der Anmeldung: 27.10.2010
(73) Patentinhaber: Spang & Brands GmbH, 61381 Friedrichsdorf (DE)
(72) Erfinder: MADER, Jürgen, 35516 Dambach (DE)
(74) Vertreter: Erb, Henning
(86) Internationale Anmeldenummer: PCT/EP2009/000340
(87) Internationale Veröffentlichungsnummer: WO 2009/100806

(56) Entgegenhaltungen:
- DE-C1- 4 425 433

## Beschreibung

Die Erfindung betrifft eine Verschlusskappe zur materialschlüssig dichten Verbindung mit einem ein flüssiges Pharmazeutikum enthaltenden Behältnis, mit einem im wesentlichen topfförmigen Außenteil aus thermoplastischem Kunststoff mit wenigstens einer stirnseitigen, dicht abgedeckten, freilegbaren Einstichöffnung und einem diese innen überdeckenden, scheiben- oder stopfenförmigen Dichtelement aus einem Weichkunststoff. Die Erfindung bezieht sich darüber hinaus auf ein Verfahren zur Herstellung einer solchen Verschlusskappe und ein mit einem flüssigen Pharmazeutikum gefülltes Behältnis aus thermoplastischem Kunststoff.

Eine derartige Verschlusskappe ist aus der DE 3 835 720 C2 bekannt. Sie wird erzeugt, indem das Material für das Dichtelement in einer Spritzgussform in das Außenteil der Verschlusskappe eingespritzt wird, so dass es nach dem Aushärten fest mit dieser verbunden ist. Da das eingespritzte Material des Dichtelements aber auch fest mit einer einstückig mit dem Außenteil geformten, abreißbaren Abdeckung der Einstichöffnung verbunden wäre, muss die Abdeckung nachträglich, zum Beispiel in Form einer aufgeklebten Metallfolie, angebracht werden, die verhältnismäßig leicht verletzt werden kann.

Weitere Verschlusskappen der genannten Art sind aus der DE 44 25 433 C1 und der DE 103 11 154 A1 bekannt. In beiden Fällen ist ein Dichtelement aus Weichkunststoff durch lokal begrenzte Erwärmung rings um die Einstichöffnung mit dem Auβenteil der Kappe verschweißt. Inzwischen hat sich jedoch herausgestellt, dass es sehr schwierig ist, in der Großserien-Produktion dauerhaft zuverlässige, dichte Schweißverbindungen zwischen dem Weichkunststoff des Dichtelements und dem Material des Außenteils der Verschlusskappe herzustellen. Immer wieder kam es in unvorhersehbarer Weise vor, dass eine Schweißverbindung schon nach dem notwendigen Sterilisiervorgang nicht mehr dicht war. Die genauen Ursachen für das Aufbrechen der Schweißverbindung sind bisher nicht bekannt. Es wird angenommen, dass die Schwierigkeiten hauptsächlich auf die sehr unterschiedlichen Materialien des Außenteils und des Dichtelements zurückzuführen sind.

Der Erfindung liegt deshalb die Aufgabe zugrunde, eine Verschlusskappe der eingangs bezeichneten Art und ein Verfahren zu ihrer Herstellung sowie ein Behältnis mit einer Verschlusskappe zu schaffen, bei der auch in der GroßserienProduktion eine zuverlässig dichte Verbindung zwischen dem Außenteil und dem Dichtelement erreicht wird, unabhängig davon, wie die äußere Abdeckung der Einstichöffnung gestaltet ist.

Vorstehende Aufgabe wird erfindungsgemäß zum einen dadurch gelöst, dass das Dichtelement durch Einspritzen in eine seinen Umfang umschließende Fassung ringsum dicht mit dieser verbunden ist und die Fassung über ihren gesamten Umfang mit dem Außenteil dicht verbunden, vorzugsweise verschweißt ist. Die Aufgabe wird zum anderen durch die Merkmale der Ansprüche 12 bzw. 13 gelöst.

Die Erfindung schlägt somit statt einer einzigen nunmehr zwei parallel angeordnete Verbindungen vor. Bei rein theoretischer Betrachtung von Zufallsfehlern wäre zu erwarten gewesen, dass sich dadurch die Fehlerwahrscheinlichkeit der Dichtung insgesamt verdoppelt. Tatsächlich wird jedoch ein höherer Sicherheitsgrad erreicht, weil jede der beiden parallel angeordneten Verbindungen mit viel größerer Sicherheit über den Sterilisiervorgang hinaus zuverlässig dicht erzeugt werden kann. Wenn, wie in bekannter Ausführung, das Außenteil der Verschlusskappe aus PE (Polyethylen) oder PP (Polypropylen) und das Dichtelement aus einem spritzgussfähigen thermoplastischen Elastomer (TPE) besteht, kann z. B. zur Erzielung einer dichten Schweißverbindung für die Fassung PE bzw. PP oder ein thermoplastisches Compoundmaterial verwendet werden, das als Haftvermittler einerseits Material, das mit dem des Dichtelements materialschlüssig verbindbar ist, und andererseits Material, das mit dem des Außenteils materialschlüssig verbindbar ist, enthält. Normalerweise ist ein solches Compoundmaterial aus Bestandteilen sowohl des Materials des Außenteils als auch des Materials des Dichtelements zusammengesetzt.

Die das Dichtelement umgebende Fassung kann an einer ihrer Stirnflächen und/oder an ihrer Umfangsfläche mit dem Außenteil verschweißt sein. In einer bevorzugten Ausführungsform der Erfindung ist das topfförmige Außenteil mit einer die Einstichöffnung umgebenden, napfförmigen Vertiefung in seinem Boden geformt, deren Querschnitt zum Querschnitt der Fassung passt, und diese ist an dem behältnisseitigen Ende mit einem Flansch geformt, der mit dem Außenteil verschweißt ist. Die Schweißverbindung kann noch dadurch verbessert werden, dass das Außenteil am Rand der napfförmigen Vertiefung mit einem diese umgebenden, von der inneren Bodenfläche abstehenden, verhältnismäßig dünnwandigen Kragen geformt ist, der mit dem an der Fassung angeformten Flansch verschweißt ist. Darüber hinaus hat diese Ausführungsform den Vorteil, dass der Kragen das Einsetzen der Fassung mit dem von ihr gehaltenen Dichtelement in das Außenteil vor dem Verschweißen erleichtert.

Wie auch von anderen Verschlusskappen bekannt, ist in der bevorzugten praktischen Ausführung das Außenteil der Kappe mit zwei Einstichöffnungen und zwei diese umgebenden, napfförmigen Vertiefungen geformt, die ineinander übergehen, wobei dann auch die Dichtelemente für die beiden Einstichöffnungen einstückig ineinander übergehend geformt sind. In diesem Fall sind die einstückig verbundenen Dichtelemente zusammen von einer einzigen Fassung umgeben. Eine alternative Ausführung sieht vor, dass das Außenteil mit zwei Einstichöffnungen und einer napfförmigen Vertiefung geformt ist, in der in einer passenden, einstückigen, gemeinsamen Fassung zwei getrennte Dichtelemente sitzen.

Da das Material für das Dichtungselement erfindungsgemäß nicht unmittelbar in das Außenteil der Verschlusskappe, sondern in eine Fassung eingespritzt ist, die anschließend in das Außenteil eingeschweißt oder ggf. eingeklebt oder mit dichtem Sitz eingeclipst wird, können die Einstichöffnungen jeweils durch einen einstückig mit dem Außenteil geformten, durch eine Sollbruchstelle begrenzten Stirnwandbereich mit angeformter Grifflasche oder -öse abgedeckt sein.

Dem vorstehend dargestellten Aufbau der neuen Verschlusskappe entspricht das zu ihrer Herstellung vorgeschlagene, neue Verfahren. Danach werden zunächst das Außenteil und die Fassung als getrennte Teile im Spritzgussverfahren erzeugt. Anschließend wird, ebenfalls in einer Spritzgussform, das Material des Dichtelements in die Fassung eingespritzt, so dass nach dem Aushärten die Fassung das dicht mit ihr verbundene Dichtelement umgibt. Schließlich wird die Fassung je nach der Form des Außenteils stirnseitig oder umfangsseitig mit dem Außenteil in bekannter Weise durch Erwärmung, die auch durch Laser erzeugt werden kann, verschweißt oder in anderer Weise verbunden.

Gegenstand der Erfindung ist schließlich auch ein Behältnis mit einem darin enthaltenen flüssigen Pharmazeutikum und mit einer erfindungsgemäßen Verschlusskappe.

Nachfolgend wird ein in der beiliegenden Zeichnung dargestelltes Ausführungsbeispiel der Erfindung näher erläutert. Es zeigen:
- Fig. 1: eine perspektivische Ansicht einer er- findungsgemäßen Verschlusskappe;
- Fig. 2: eine Draufsicht auf die Verschlusskappe nach Fig. 1;
- Fig. 3: einen Querschnitt durch die Verschluss- kappe gemäß Schnittlinie A-A in Fig. 2;
- Fig. 4: einen Querschnitt durch die Verschluss- kappe nach Schnittlinie B-B in Fig. 2;
- Fig. 5: eine Ansicht von unten auf das Außen- teil der Verschlusskappe nach Fig. 1 und 2 vor dem Einsetzen des Dichtele- ments;
- Fig. 6: einen Querschnitt des Außenteils der Verschlusskappe nach Fig. 5 gemäß Schnittlinie A-A in Fig. 2;
- Fig. 7: einen Querschnitt des Außenteils der Verschlusskappe nach Fig. 5 gemäß Schnittlinie B-B in Fig. 2;
- Fig. 8: eine Seitenansicht einer Fassung zur Aufnahme des Dichtelements der Ver- schlusskappe;
- Fig. 9: eine Draufsicht auf die Fassung nach Fig. 8 nach dem Einspritzen und Aushär- ten des Dichtelements, und
- Fig. 10: einen Längsschnitt durch die Fassung und das darin mit dichter Verbindung sitzende Dichtelement.

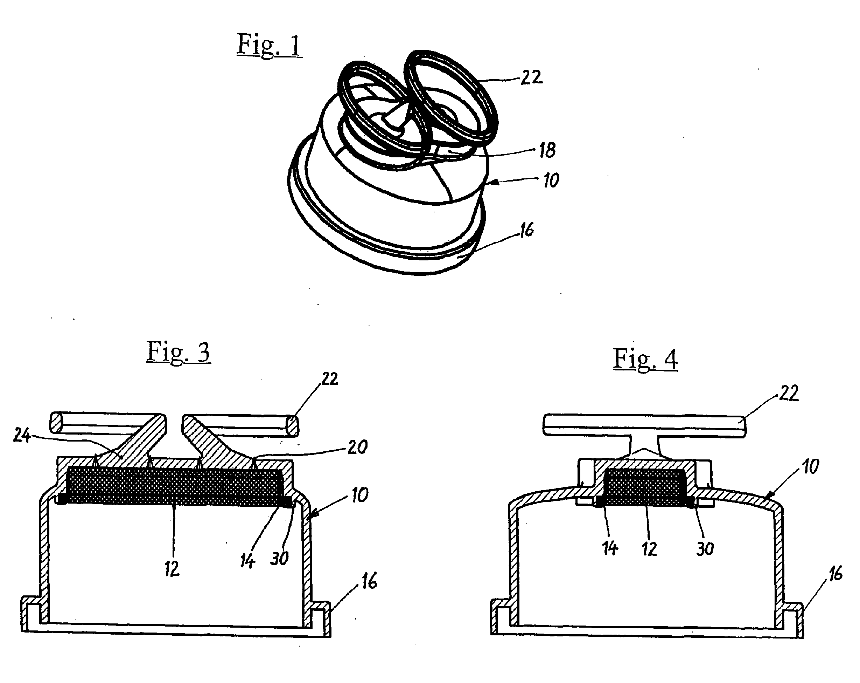

Die in den Fig. 1 bis 4 gezeigte Verschlusskappe besteht aus einem im wesentlichen topfförmigen Außenteil 10 z. B. aus Polyethylen (PE) oder Polypropylen (PP), einem Dichtelement 12 aus einem thermoplastischen Elastomer (TPE) sowie einer das Dichtelement 12 am Umfang umschließenden Fassung 14 aus PE bzw. PP oder einem Compoundmaterial, das Bestandteile sowohl des Materials des Außenteils 10 als auch des Materials des Dichtelements 12 enthält.

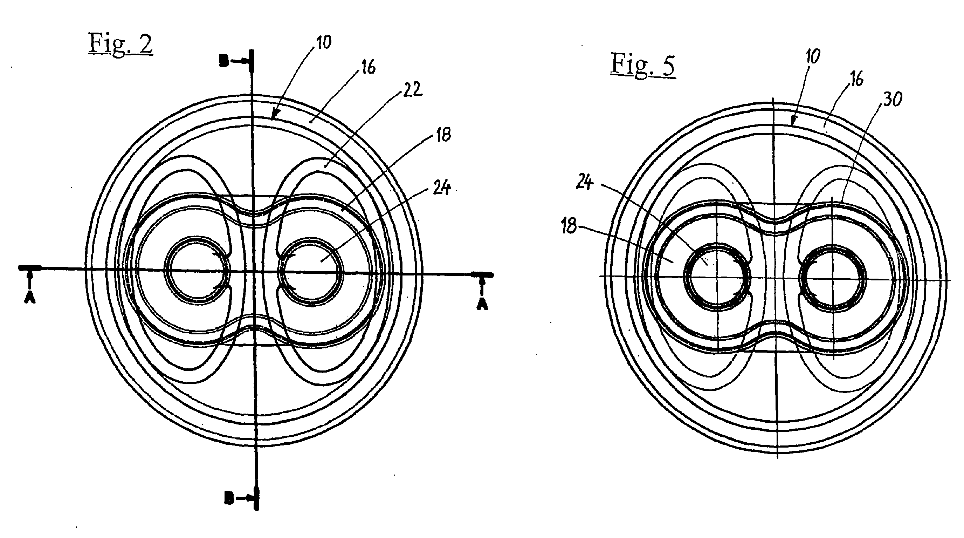

Das Außenteil 10 hat an seinem unteren, offenen Ende einen Flanschrand 16, der in bekannter Weise mit dem entsprechend ausgebildeten Hals eines ein Pharmazeutikum enthaltenden Behältnisses ringsum dicht zu verschweißen ist. In den Boden des topfförmigen Außenteils 10 ist eine napfförmige Vertiefung 18 eingeformt, welche die aus den Fig. 2 und 5 ersichtliche, längliche Form zweier ineinander übergehender zylindrischer Vertiefungen mit einer Taille im mittleren Übergangsbereich hat. Selbstverständlich könnte der Übergangsbereich auch geradlinig tangential oder die gesamte Vertiefung einen im wesentlichen elliptischen Querschnitt haben. Die gezeigte Form mit Taille ist im Hinblick auf eine Minimierung der Querschnittsfläche gewählt worden. Dadurch wird auch die Masse des in der napfförmigen Vertiefung 18 sitzenden Dichtelements 12 reduziert.

Das Material des Dichtelements 12 ist bei der dargestellten Verschlusskappe allerdings nicht direkt in das Außenteil 10 eingespritzt worden. Dies hätte dazu geführt, dass es sich beim Einspritzvorgang nicht nur am Umfang, sondern auch stirnseitig über die gesamte Bodenfläche des topfförmigen Außenteils 10 fest mit dieser verbunden hätte. Dadurch wäre verhindert worden, dass man, wie bei der gezeigten Verschlusskappe, jeweils Teile der den Boden bildenden, stirnseitigen Endwand, die jeweils durch eine Sollbruchstelle 20 umgrenzt sind, durch Herausreißen abtrennen kann, um Einstechöffnungen für kanülenförmige Anschlußenden von Infusionsschläuchen freizulegen. Im Ausführungsbeispiel hat die Verschlusskappe zwei durch Sollbruchstellen 20 umgrenzte Einstichöffnungen, die einzeln durch Ziehen an ovalen Ösen 22 freigelegt werden können, die jeweils an einen von einer ringförmig geschlossenen Sollbruchlinie 20 umgrenzten Bereich 24 der Endwand angeformt sind.

Damit das Dichtelement 12 nicht fest an den Endwandbereichen 24 des Außenteils 10 haftet, ist es getrennt vom Außenteil 10 hergestellt und danach in die napfförmige Vertiefung 18 eingesetzt und darin befestigt worden.

Weil sich das aus TPE bestehende Dichtelement 12, wie bereits eingangs erwähnt, nicht zuverlässig mit dem aus PE oder PP bestehenden Außenteil der Verschlusskappe verschweißen lässt, wird es erfindungsgemäß in einer Spritzgußform in die zuvor im Spritzgußverfahren geformte Fassung 14 eingespritzt, wobei an der Innenfläche der Fassung 14 eine feste und dichte Verbindung mit dem Dichtelement 12 zustande kommt, wenn die Fassung 14, wie bei der EP 0 364 783 B1 das Außenteil, aus PE oder PP und das Dichtelement 12 aus einem TPE besteht. Um die dichte materialschlüssige Verbindung zwischen der Fassung 14 und dem Dichtelement 12 noch zu verbessern, kann die Fassung 14 aus einem spritzgußfähigen Compundmaterial geformt werden, das, ebenso wie das Außenteil 10, aus PE bzw. PP besteht und zusätzlich als Haftvermittler Bestandteile des Materials des Dichtelements aufweist.

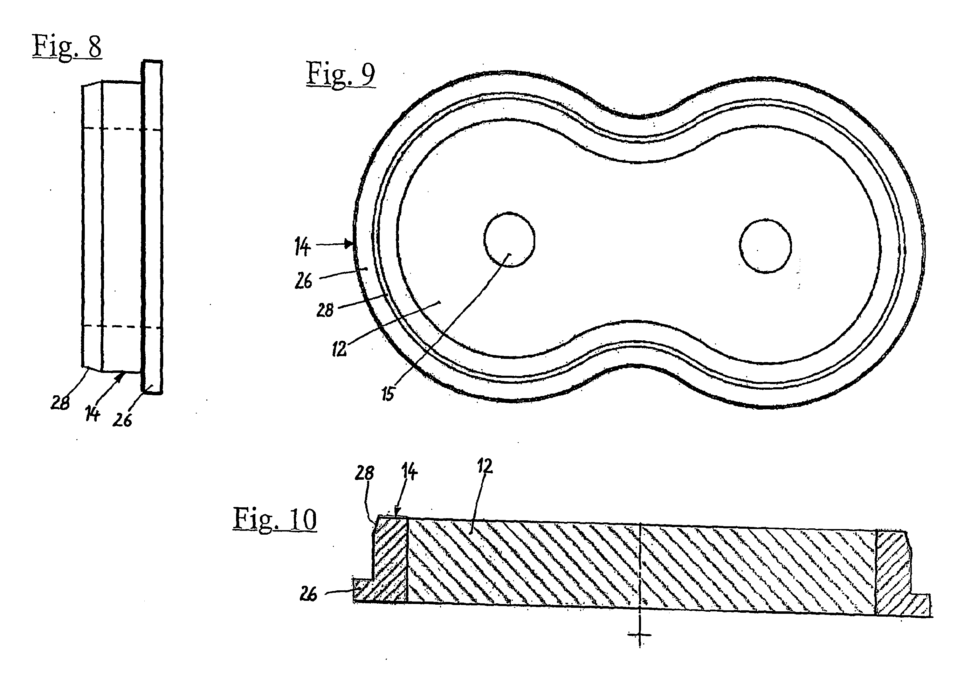

Vorzugsweise hat die Fassung 14 die in den Fig. 8 und 9 gezeigte, aus zwei in einer Taille ineinander übergehenden, runden Ringen bestehende Form und den aus Fig. 10 ersichtlichen Querschnitt mit einem äußeren Flansch 26 an dem einen Ende und einer Fase 28 an dem anderen Ende. Die Innenfläche der Fassung 14 ist in Fig. 10 axial geradlinig gezeigt, kann aber auch z. B. mit axialen oder radialen Rippen oder Nuten geformt sein, wenn sich dies zum festen Halt des Dichtelements 12 als notwendig erweisen sollte.

Im Ausführungsbeispiel schließt das Dichtelement 12 stirnseitig bündig mit den Stirnflächen der Fassung 14 ab und kann gemäß Fig. 9 im Zentrum der Einstichöffnungen jeweils mit einer Mulde 15 geformt sein, die das Einstechen einer Kanüle erleichtert. Das Dichtelement 12 könnte aber auch so geformt werden, dass es auf der Seite, wo sich die Fase 28 befindet, ein wenig über die Stirnfläche der Fassung 14 vorsteht und im fertigen Zustand der Verschlußkappe jeweils rings um die Sollbruchstellen 20 mit leichter Vorspannung am Boden der Vertiefung 18 anliegt.

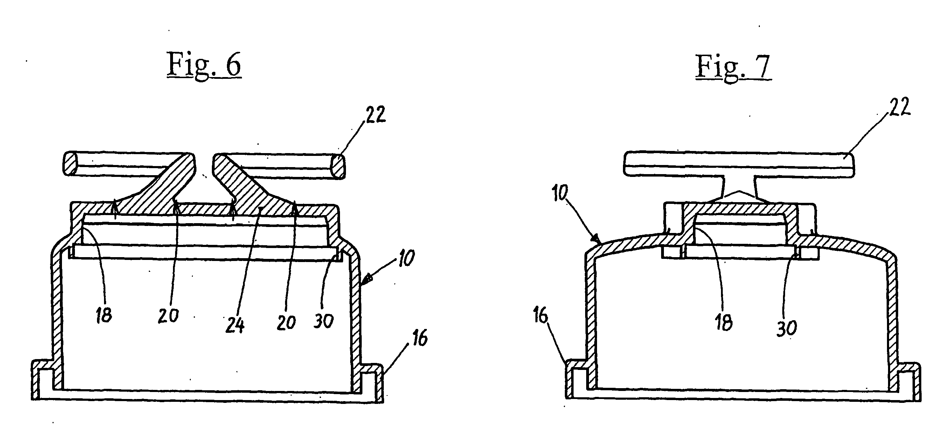

Wie am besten aus den Fig. 6 und 7 ersichtlich, ist das Außenteil 10 rings um die napfförmige Vertiefung 18 mit einem relativ dünnwandigen, sich axial zum offenen Ende hin erstreckenden Kragen 30 geformt. Der Abstand des Kragens 30 von der äußeren Randkante der Vertiefung 18 entspricht der Breite des Flanschs 26 der Fassung 14. Die äußeren Umrisse einerseits der Fassung 14 mit dem Flansch 26 und andererseits der Vertiefung 18 und des Kragens 30 sind so gewählt, dass der Flansch 26 in den Kragen 30 und der übrige axiale Teil der Fassung 14 mit der am vorderen Ende angebrachten Fase 28 in die entsprechend geformte, sich zum Ende hin verjüngende Vertiefung 18 passt. Dabei bietet die Fase 28 den Vorteil der leichteren Einführung der Fassung 14 in die Vertiefung 18 und eines festen und entsprechend dichten Sitzes in dem verjüngten Ende der Vertiefung 18. Auch der Kragen 30 fördert die Positionierung der Fassung 14 bei der Montage des Dichtelements 12, und er erleichtert ein dichtes Verschweißen der Fassung 14 mit dem Außenteil 10 der Verschlusskappe mittels eines passend geformten Heizelements, das den dünnwandigen Kragen 30 und den ebenfalls verhältnismäßig dünnwandigen Flansch 26 in kurzer Zeit auf die erforderliche Schweißtemperatur erhitzt, so dass rings um die Fassung 14 eine dichte Schweißverbindung zustande kommt.

Die Erwärmung eines Bereichs der Fassung 14 und eines anliegenden Bereichs des Außenteils 10 an der genannten oder einer anderen Stelle der Fassung 14 könnte auch auf andere Weise erfolgen, z. B. durch Laser, ggf. bei einem lichtdurchlässigen Außenteil 10 auch von außen durch dessen Wand hindurch. Auch eine Verschweißung der Fassung 14 mit dem Außenteil 10 durch Ultraschallschweißung ist möglich, wenn durch die Gestaltung der Sollbruchstellen 20 sichergestellt ist, dass sie durch die Schwingungen beim Ultraschallschweißen nicht undicht werden.

## Patentansprüche

1. Verschlusskappe zur materialschlüssig dichten Verbindung mit einem ein flüssiges Pharmazeutikum enthaltenden Behältnis, mit einem im wesentlichen topfförmigen Außenteil (10) aus thermoplastischem Kunststoff mit wenigstens einer stirnseitigen, dicht abgedeckten, freilegbaren Einstichöffnung (20, 24) und einem diese innen überdeckenden, scheiben- oder stopfenförmigen Dichtelement (12) aus einem Weichkunststoff, **dadurch gekennzeichnet, dass** das Dichtelement (12) durch Einspritzen in eine seinen Umfang umschließende Fassung (14) ringsum dicht mit dieser verbunden ist und die Fassung (14) über ihren gesamten Umfang mit dem Außenteil (10) dicht verbunden ist.

2. Verschlusskappe nach Anspruch 1, **dadurch gekennzeichnet, dass** die Fassung (14) mit dem Außenteil (10) dicht verschweißt ist.

3. Verschlusskappe nach Anspruch 1 oder 2, **dadurch gekennzeichnet, dass** das Außenteil (10) aus PE (Polyethylen) oder PP (Polypropylen) und das Dichtelement (12) aus einem spritzgussfähigen thermoplastischen Elastomer (TPE) besteht.

4. Verschlusskappe nach Anspruch 2 oder 3, **dadurch gekennzeichnet, dass** die Fassung (14) aus PE, PP oder einem thermoplastischen Compoundmaterial besteht, das einerseits Material, das mit dem des Dichtelements (12) materialschlüssig verbindbar ist, und andererseits Material, das mit dem des Außenteils (10) verschweißbar ist, enthält.

5. Verschlusskappe nach Anspruch 4, **dadurch gekennzeichnet, dass** das Compoundmaterial Bestandteile sowohl des Materials des Außenteils (10) als auch des Materials des Dichtelements (12) enthält.

6. Verschlusskappe nach einem der vorhergehenden Ansprüche, **dadurch gekennzeichnet, dass** die Fassung (14) an einer ihrer Stirnflächen und/oder an ihrer Umfangsfläche mit dem Außenteil (10) verschweißt ist.

7. Verschlusskappe nach Anspruch 6, **dadurch gekennzeichnet, dass** das topfförmige Außenteil (10) mit einer die Einstichöffnung (20, 24) umgebenden, napfförmigen Vertiefung (18) in seinem Boden geformt ist, deren Querschnitt zum Querschnitt der Fassung (14) passt, und dass die Fassung (14) an dem behältnisseitigen Ende mit einem Flansch (26) geformt ist, der mit dem Außenteil (10) verschweißt ist.

8. Verschlusskappe nach Anspruch 7, **dadurch gekennzeichnet, dass** das Außenteil (10) am Rand der napfförmigen Vertiefung (18) mit einem diese umgebenden, von der inneren Bodenfläche abstehenden Kragen (30) geformt ist, der mit dem an der Fassung (14) angeformten Flansch (26) verschweißt ist.

9. Verschlusskappe nach Anspruch 7 oder 8, **dadurch gekennzeichnet, dass** das Außenteil (10) mit zwei Einstichöffnungen (20, 24) und zwei diese umgebenden, napfförmigen Vertiefungen (18) geformt ist, die ineinander übergehen, und dass die Dichtelemente (12) für die beiden Einstichöffnungen (20, 24) einstückig ineinander übergehend geformt und zusammen von einer einzigen Fassung (14) umgeben sind.

10. Verschlusskappe nach Anspruch 7 oder 8, **dadurch gekennzeichnet, dass** das Außenteil (10) mit zwei Einstichöffnungen (20, 24) und einer napfförmigen Vertiefung (18) geformt ist, in der in einer einstückigen, gemeinsamen Fassung (14) zwei getrennte Dichtelemente (12) sitzen.

11. Verschlusskappe nach einem der vorhergehenden Ansprüche, **dadurch gekennzeichnet, dass** die Einstichöffnung bzw. Einstichöffnungen (20, 24) jeweils durch einen einstückig mit dem Außenteil (10) geformten, durch eine Sollbruchstelle (20) begrenzten Stirnwandbereich (24) mit angeformter Grifflasche oder -öse (22) abgedeckt sind.

12. Verfahren zur Herstellung einer Verschlusskappe zur materialschlüssig dichten Verbindung mit einem ein flüssiges Pharmazeutikum enthaltenden Behältnis, mit einem im wesentlichen topfförmigen, im Spritzgußverfahren geformten Außenteil (10) aus thermoplastischem Kunststoff mit wenigstens einer stirnseitigen, dicht abgedeckten, freilegbaren Einstichöffnung (20, 24) und einem diese innen überdeckenden, scheiben- oder stopfenförmigen Dichtelement (12) aus einem Weichkunststoff, **dadurch gekennzeichnet, dass** im Spritzgußverfahren eine Fassung (14) für das Dichtelement geformt wird, dann in einer Spritzgußform das Material des Dichtelements (12) in die Fassung (14) eingespritzt und anschließend die Fassung (14) stirnseitig oder umfangsseitig über ihren gesamten Umfang mit dem Außenteil (10) dicht verbunden, z. B. verschweißt wird.

13. Mit einem flüssigen Pharmazeutikum gefülltes Behältnis aus thermoplastischem Kunststoff, **dadurch gekennzeichnet, dass** es mit einer Verschlusskappe nach einem der Ansprüche 1 bis 11 zu einer Einheit dicht verschweißt ist.

## Claims

1. A closure cap for tight connection in material-locking fashion to a container containing a liquid pharmaceutical, having an essentially cup-shaped outer part (10) of thermoplastic plastic with at least one needle-access opening (20, 24) on the face end that is tightly covered and can be exposed, and having a disklike or stopperlike sealing element (12) covering the needle-access opening on the inside and comprising a soft plastic, **characterized in that** the sealing element (12), by injection into a mounting (14) surrounding its circumference, is connected tightly all the way around to the mounting, and the mounting (14) is connected tightly over its entire circumference to the outer part (10).

2. The closure cap as defined by claim 1, **characterized in that** the mounting (14) is tightly welded to the outer part (10).

3. The closure cap as defined by claim 1 or 2, **characterized in that** the outer part (10) comprises PE (polyethylene) or PP (polypropylene), and the sealing element (12) comprises an injection-moldable thermoplastic elastomer (TPE).

4. The closure cap as defined by claim 2 or 3, **characterized in that** the mounting (14) comprises PE, PP, or a thermoplastic compound material, which contains on the one hand material that is connectable in material-locking fashion to the material of the sealing element (12), and on the other, material that is weldable to the material of the outer part (10).

5. The closure cap as defined by claim 4, **characterized in that** the compound material contains ingredients of both the material of the outer part (10) and of the material of the sealing element (12).

6. The closure cap as defined by one of the foregoing claims, **characterized in that** the mounting (14) is welded on one of its end faces and/or on its circumferential surface to the outer part (10).

7. The closure cap as defined by claim 6, **characterized in that** the cup-shaped outer part (10) is shaped with a bowl-shaped indentation (18) in its bottom, the indentation surrounding the needle-access opening (20, 24), and the cross section of the indentation fits the cross section of the mounting (14); and that the mounting (14), on the end toward the container, is shaped with a flange (26) that is welded to the outer part (10).

8. The closure cap as defined by claim 7, **characterized in that** the outer part (10), on the periphery of the bowl-shaped indentation (18), is shaped with a collar (30), surrounding the indentation and protruding from the inner bottom face, which collar is welded to the flange (26) that is integrally formed onto the mounting (14).

9. The closure cap as defined by claim 7 or 8, **characterized in that** the outer part (10) is shaped with two needle-access openings (20, 24) and with two bowl-shaped indentations (18) surrounding them, which merge with one another; and that the sealing elements (12) for the two needle-access openings (20, 24) are shaped merging in one piece with one another and together are surrounded by a single mounting (14).

10. The closure cap as defmed by claim 7 or 8, **characterized in that** the outer part (10) is shaped with two needle-access openings (20, 24) and one bowl-shaped indentation (18), in which indentation, two separate sealing elements (12) are seated in the same one-piece mounting (14).

11. The closure cap as defined by one of the foregoing claims, **characterized in that** the needle-access opening or needle-access openings (20, 24) are each covered by an end wall region (24), shaped in one piece with the outer part (10) and defined by a rated breaking line (20), with an integrally formed-on grip tab or grip eyelet (22).

12. A method for producing a closure cap for tight connection in material-locking fashion to a container containing a liquid pharmaceutical, having an essentially cup-shaped outer part (10) of thermoplastic material, formed by an injection molding process, with at least one needle-access opening (20, 24) on the face end that is tightly covered and can be exposed, and having a disklike or stopperlike sealing element (12) covering the needle-access opening on the inside and comprising a soft plastic, **characterized in that** by an injection molding process, a mounting (14) for the sealing element is formed; then in an mold, the material of the sealing element (12) is injected into the mounting (14); and then the mounting (14), on its face end or circumference, is tightly connected, for instance welded, over its entire circumference to the outer part (10).

13. A container of thermoplastic material, filled with a liquid pharmaceutical, **characterized in that** it is tightly welded to a closure cap as defined by one of claims 1 through 11, forming a unit.

## Revendications

1. Capot de fermeture servant à l'assemblage étanche par concordance des matériaux d'un récipient contenant un produit pharmaceutique liquide, avec une partie extérieure essentiellement en forme de godet (10) à base d'une matière thermoplastique avec au moins une ouverture pour perforation (20, 24) du côté frontal recouverte hermétiquement et pouvant être découverte et avec un élément d'étanchéité (12) constitué d'une matière plastique souple en forme de rondelle ou de tampon recouvrant cette ouverture de l'intérieur, **caractérisé en ce que** l'élément d'étanchéité (12) est joint tout autour hermétiquement avec une monture (14) par injection dans cette monture (14) l'enveloppant dans sa circonférence et que la monture (14) est jointe hermétiquement sur tout son pourtour avec la partie extérieure (10).

2. Capot de fermeture selon la revendication 1, **caractérisé en ce que** la monture (14) est soudée hermétiquement à la partie extérieure (10).

3. Capot de fermeture selon la revendication 1 ou 2, **caractérisé en ce que** la partie extérieure (10) est constituée de PE (polyéthylène) ou de PP (polypropylène) et que l'élément d'étanchéité (12) est élaboré à base d'un élastomère thermoplastique (TPE), pouvant être moulé par injection sous pression.

4. Capot de fermeture selon la revendication 2 ou 3, **caractérisé en ce que** la monture (14) est constituée de PE, PP ou d'un composite thermoplastique qui contient d'une part un matériau pouvant être assemblé par concordance à celui de l'élément d'étanchéité (12) et d'autre part un matériau pouvant être soudé à la partie extérieure (10).

5. Capot de fermeture selon la revendication 4, **caractérisé en ce que** le composite contient des constituants tant du matériau de la partie extérieure (10) que du matériau de l'élément d'étanchéité (12).

6. Capot de fermeture selon l'une des revendications précédentes, **caractérisé en ce que** la monture (14) est soudée au niveau de l'une de ses faces frontales et/ou de sa surface circonférentielle à la partie extérieure (10).

7. Capot de fermeture selon la revendication 6, **caractérisé en ce que** la partie extérieure en forme de godet (10) est façonnée dans son fond avec une cavité (18) en forme de cuvette entourant l'ouverture pour perforation (20, 24), dont la section correspond à la section de la monture (14) et que la monture (14) est façonnée à l'extrémité tournée vers le récipient avec une bride à collerette (26) qui est soudée à la partie extérieure (10).

8. Capot de fermeture selon la revendication 7, **caractérisé en ce que** la partie extérieure (10) est façonnée au bord de la cavité (18) en forme de cuvette avec un rebord (30) qui l'entoure en saillie par rapport à la surface intérieure du fond et qui est soudé à la bride à collerette (26) moulée à la monture (14).

9. Capot de fermeture selon la revendication 7 ou 8, **caractérisé en ce que** la partie extérieure (10) est façonnée avec deux ouvertures pour perforation (20, 24) et avec deux cavités (18) en forme de cuvette les entourant qui se confondent et que les éléments d'étanchéité (12) des deux ouvertures pour perforation (20, 24) sont façonnés d'un bloc fusionnant l'un dans l'autre et entourés conjointement d'une seule monture (14).

10. Capot de fermeture selon la revendication 7 ou 8, **caractérisé en ce que** la partie extérieure (10) est façonnée avec deux ouvertures pour perforation (20, 24) et une cavité (18) en forme de cuvette, dans laquelle deux éléments d'étanchéité (12) séparés sont logés à l'intérieur d'une monture commune (14) d'un seul tenant.

11. Capot de fermeture selon l'une des revendications précédentes, **caractérisé en ce que** l'ouverture pour perforation ou les ouvertures pour perforation (20, 24) sont respectivement recouvertes par une zone de la paroi frontale (24), façonnée d'un seul tenant avec la partie extérieure (10) mais limitée par une ligne de rupture prévue (20) et munie d'une languette ou d'un oeillet de préhension (22).

12. Procédé pour fabriquer un capot de fermeture servant à l'assemblage étanche par concordance des matériaux d'un récipient contenant un produit pharmaceutique liquide, moulage par injection, essentiellement en forme de godet (10) à base d'une matière thermoplastique avec au moins une ouverture pour perforation (20, 24) du côté frontal, recouverte hermétiquement et pouvant être découverte, et avec un élément d'étanchéité (12) constitué d'une matière plastique souple en forme de rondelle ou de tampon recouvrant cette ouverture de l'intérieur, **caractérisé en ce qu'**une monture (14) pour l'élément d'étanchéité est façonnée par le procédé de moulage par injection, que le matériau de l'élément d'étanchéité (12) est ensuite injecté dans la monture (14) à l'intérieur d'un moule pour injection et qu'enfin la monture (14) est reliée hermétiquement, par example soudée, sur sa face frontale ou au niveau de sa circonférence sur tout son pourtour à la partie extérieure (10).

13. Récipient en matière thermoplastique, rempli d'un produit pharmaceutique liquide, **caractérisé en ce qu'**il est soudé de manière étanche à un capot de fermeture conformément à l'une des revendications de 1 à 11 pour former une unité.
